# EUROPEAN PATENT APPLICATION

(11) **EP 2 368 492 A1**
(43) Date of publication of application: **28.09.2011**
(21) Application number: 10157807.8
(22) Date of filing: 25.03.2010
(51) Int. Cl.: A61B 5/024, A61B 5/11

(54) **A method for estimating parameters indicative of a heart performance, a radar system and a computer program product**

(71) Applicant: Nederlandse Organisatie voor Toegepast -Natuurwetenschappelijk Onderzoek TNO, 2628 VK Delft (NL)
(72) Inventor: van Dorp, Philip, 2628 VK delft (NL)
(74) Representative: Hatzmann, Martin

(57) **Abstract**

The invention relates to a method for estimating parameters indicative of a heart performance. The method comprises the step of receiving a radar signal reflected by a human heart. Further, the method comprises the steps of generating synthetic radar data using a parametrized heart model. The method also comprises the step of optimizing at least one parameter of the heart model by fitting the received radar signal with the generated synthetic radar data.

## Description

The invention relates to a method for estimating parameters indicative of a heart performance.

It is known to monitor the heart performance using electro optical systems and/or acoustical systems. Such systems include sensors that are brought into contact with the skin of a person to be monitored.

It is an object of the invention to provide a method according to the preamble for estimating parameters indicative of a heart performance that is relatively comfortable for the person that is subjected to the measurement. Thereto, the method according to the invention comprises the steps of receiving a radar signal reflected by a human heart, generating synthetic radar data using a parametrized heart model, and optimizing at least one parameter of the heart model by fitting the received radar signal with the generated synthetic radar data.

By using radar data, a non-contact measurement can be performed, so that the person that is subjected to the measurement is not required to undress, thereby rendering the technique easily applicable, e.g. when patients have to be monitored during a relatively long period. Due to the non-contact aspect of the measurement, the method is relatively comfortable for the person involved. In addition, by fitting the received radar signal to synthetic radar data that has been generated based on a parametrized heart model, a priori information is advantageously incorporated in the measuring process, thereby rendering the method efficient, stable, fast and accurate.

Further, the invention relates to a radar system.

Additionally, the invention relates to a computer program product. A computer program product may comprise a set of computer executable instructions stored on a data carrier, such as a flash memory, a CD or a DVD. The set of computer executable instructions, which allow a programmable computer to carry out the method as defined above, may also be available for downloading from a remote server, for example via the Internet.

Other advantageous embodiments according to the invention are described in the following claims.

By way of example only, embodiments of the present invention will now be described with reference to the accompanying figures in which
Fig. 1 shows a schematic view of a radar system according to the invention;
Fig. 2a shows a measured heart time signal;
Fig. 2b shows an estimated heart time signal;
Fig. 3a shows a measured heart spectrogram signal;
Fig. 3b shows an estimated heart spectrogram signal; and
Fig. 4 shows a flow chart of steps that are performed in a method according to the invention.

The figures are merely schematic views of preferred embodiments according to the invention. In the figures, the same reference numbers refer to equal or corresponding parts.

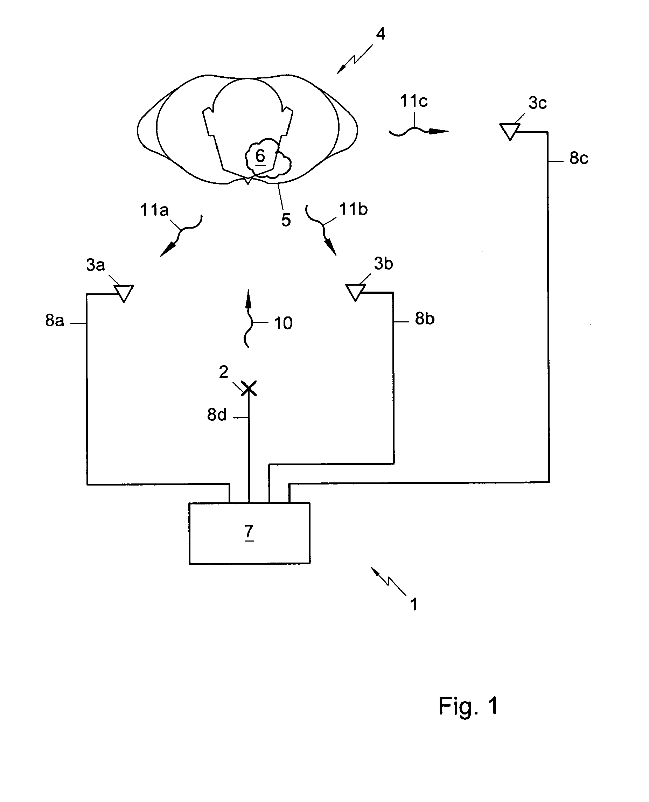

Figure 1 shows a schematic view of a radar system 1 according to the invention. The radar system 1 comprises a transmitter 2 for transmitting radar signals 10 and a multiple number of receivers 3a-c positioned at around a person 4 to be scanned. During use of the radar system 1, radar signals 10 are transmitted to the person, in particular to the person's chest 5 including the heart 6, reflecting the transmitted signals 10 as reflected signals. A part of the reflected signals 11a-c is received by corresponding receivers 3a-c.

The radar system 1 further comprises a processing unit 7 that is arranged for performing a number of steps. The processing unit 7 is connected to the transmitter 2 and to the receivers 3a-c via data lines 8a-d. The transmitter 2 and the receivers 3a-c form a radar equipment for transmitting a radar signal 10 towards a human heart 6 and for receiving a radar signal 11 reflected by the human heart 6.

The frequency of the used radar signals is chosen such that the signals may penetrate into the human body. As an example, the signal frequency is in a frequency ranges between circa 2.4 GHz and circa 5.6 GHz. It is noted that the above-mentioned frequency range is purely illustrative and no limiting the claimed invention to such frequency range. As an example, also signal frequencies below 2.4 GHz can in principle be applied.

The radar signals 10, 11 are of the so-called frequency modulated continuous wave signal (FMCW) type. Thereto, a continuous wave signal is modulated in frequency to produce a linear sweep that is radiated towards the person's heart 6. The reflected and received radar signal 11 is mixed with the transmitted signal to produce a beat frequency signal that is proportional with a round trip time covering the distance from the transmitter to the heart added to the distance from the heart to the receiver 3. The beat signal includes specific patterns that are indicative for the heart performance, e.g. in terms of Doppler distribution and velocity distribution. It is noted that, in principle, also other radar signals can used, e.g. radar pulse signals.

The processing unit 7 is arranged for generating synthetic radar data using a parametrized heart model, and for optimizing at least one parameter of the heart model by fitting a received radar signal, reflected by a human heart, with the generated synthetic radar data.

The step of generating synthetic radar data includes computing radar data based on a parametrized heart model. The parametrized heart model is e.g. a geometric heart model such as an ellipsoid heart model neglecting a specific heart wall change during the heart cycle, or a heart surface variation model including typical surface variations of the heart during the heart cycle based on a set of real time MRI images. Both models represent the heart by the outer heart wall. Apparently, also more refined heart models can be applied, such as a heart model including the action of heart valves or a heart model including a representation of blood perfusion. Further, also other data can be included in the parametrized heart model, such as location information of the heart, e.g. using a grid, and/or known EM material properties of the heart and surrounding tissue, such as the electrical permittivity.

In general, a parametrized heart model is characterized by a single or a multiple number of parameters having numerical values. Examples of such parameters are a frequency, an amplitude and/or a phase of a heart beating rhythm. Other examples of such parameters include geometrical dimensions of the heart, e.g. the heart volume, either averaged over time or instantaneous. Also other dynamic parameters can characterize the heart model, such as heart valve parameters or an actual blood perfusion.

Assuming an initial value for parameters characterizing a parametrized heart volume, a particular model of a human heart is obtained that can be used for computing synthetic radar data that would be received by the receiver(s) if the model is identical to the real heart.

The step of optimizing at least one parameter of the heart model includes fitting a received, reflected radar signal with the generated synthetic radar data.

The fitting process can be performed either in the time domain or in a computation domain, such as a spectral domain. Further, the optimization can include solving a least squares function or other optimization function. When the fitting process is performed in the spectral domain, a bandwidth extrapolation spectrogram processing step can be performed, including estimating signal domain parameters by finding the best fitted parameters of an analytical function. Said function provides information outside the range of the measurements and can be subjected to a Fourier transform thus providing a higher spectral resolution.

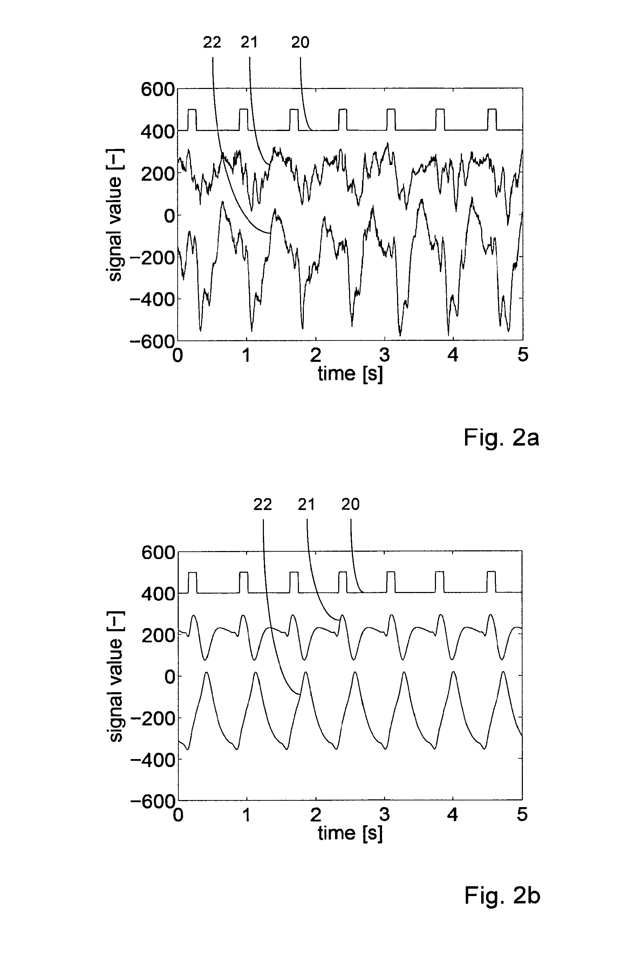

Figures 2a and 2b show a measured heart time signal and an estimated heart time signal, respectively. In Fig. 2a, b an upper signal 20 denotes an ECG signal, while the two lower signals 21, 22 denote the real and imaginary part of the heart signal as function of time.

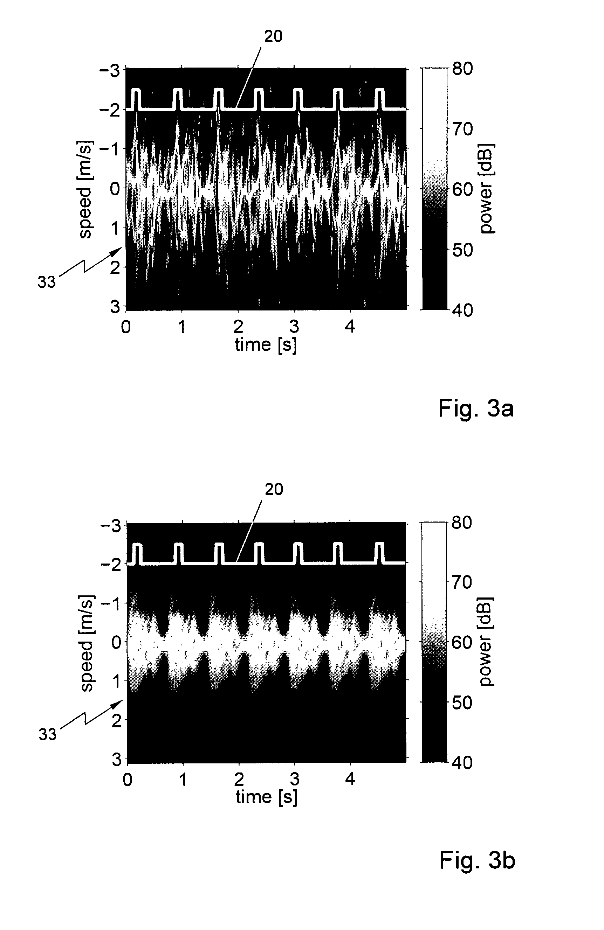

Similarly, Figures 3a and 3b show a measured heart spectrogram signal and an estimated heart spectrogram signal, respectively. In Fig. 3a, b an upper signal 20 denotes an ECG signal, while the lower signal 33 denote the spectrogram signal as function of time.

The heart frequency grid lays in the interval 0.7-1.7 Hz. The heart frequency estimated of the time signal is approximately fh =1.5 Hz and phase *t*0= 0.88. The frequency-phase fit function shows two low minima one is the normal fit and the other the conjugate fit. The amplitudes which belong to these two minima are high compared with the other frequency-phase combination on the grid. The volume fit function with fixed frequency and phase shows one strong minimum around *υh* = 600 cm3 that is selected and estimated with linear interpolation. The estimated heart signal shows strong similarities with the measured time signal. The measured signal shows high frequency fluctuations not present in the modelled signal.

The spectrogram function has approximately 0.128 s window length, the update rate is 125 Hz and BWE expansion factor of 12. The stationary background and respiration have been removed with a high pass filter with transition frequency of 0.7 Hz. The example fit function has a two step minimization. First the minimization of the fit function with fixed volume followed with a minimization of the fit function with fixed heart frequency and phase.

The spectrogram shows speed variations from -1.5 m/s to 1.5 m/s with higher signal density for positive frequencies than negative frequencies. The frequency-phase fit function shows one clear minimum with fh = 1.5 Hz frequency and phase *t*0 =0.80. The volume error fit function shows one minimum around 700 cm3. The measured spectrogram shows similarities and differences with the estimated spectrogram. The shapes are similar but the measured spectrogram is broader. The estimated spectrogram shows symmetric speed behavior while the measured spectrogram is not symmetric. The measured spectrogram and the simulated spectrogram show maximum speed deviation at the upward transition of the ECG signal. The measured spectrogram and modelled spectrogram show shape similarities. The measured signal shows high speed fluctuations not present in the modelled signal. The estimated frequency and phase of the inverse time and inverse frequency methods are comparable. Both examples show that the simulated heart signals have strong similarities with the measurements. The measurements show high speed fluctuations not present in the simulated signal. The differential signals show in the time domain peaks with approximately 0.2 s delay in comparison to the ECG signal. Similarly, the differential signals show in the spectral domain high speed fluctuations with 0.2s compared with the ECG signal.

When computing the heart volume, the following is found. In the inverse time approach, the fit functions show smaller minimum fit and smooth fit in the non respiration intervals compared with the respiration intervals. The measurements contain some irregularities due to shocking respiration or hiccups, for example, at 120 s. These irregularities introduce high frequency variations that are not filtered out by the high pass filter. In most of the cases, these irregularities have little influence onto the fit function but not during a hiccup. The estimated volumes in the respiration intervals are higher than the volumes in the non respiration intervals. The non respiration intervals show a small ripple onto the estimated volume factor.

In the inverse frequency approach, the estimated volumes are greater in respiration intervals than in non respiration intervals. The error fit functions show less distinction between respiration intervals and non respiration intervals compared with the inverse time approach. The estimated volumes show outliers, which indicate a sensitive fit function. The estimated volume is greater than with the inverse time method. Analysis of the measurements shows differences in the estimated parameters in the respiration and non respiration interval.

According to an aspect of the invention, a model-based approach is applied to estimate the heart parameters with FMCW radar. The model-based approach simulates the heart response with MRI data of the moving heart wall. The model may contain the heart frequency and heart volume (scale factor). The propagation into the human body can be simulated with a multilayered dielectric structure. The simulated FMCW radar response is fitted onto real measurements. These fits have been carried out onto time slices and spectrograms of the heart responses called the inverse time method and inverse frequency method. The constant background noise and respiration signals can be removed with a high pass filter. The spectrograms can be calculated with Bandwidth Extrapolation algorithm in order to improve the speed resolution.

The respiration frequency and power can be estimated with Quadratically Interpolated Fast Fourier Transform (QIFFT) and/or centroid method applied onto the spectrum. Classification between respiration and non respiration with a Support Vector Classifier and respiration frequency and power parameters gives 3% error in a measurement analysis.

A high pass filtering can separate stationary and respiration signals from heart signals. Disturbances with high frequencies for examples hiccups remain in the signal and disturbed the heart signal. Validation of ten heart frequency estimation methods against ECG frequency reference shows that both the spectrogram and inverse method improve the heart frequency estimation with respect to existing methods (Droitcour). The frequency bias is negligible the standard deviation is less than 0.06 Hz in a measurement.

The estimated heart time signal with inverse time method and estimated heart spectrogram with inverse frequency method give a realistic look alike of the measurements. The inverse time volume estimate differs with the expected adult human heart volume the minimum fit function deviates from the expected minimum. The inverse frequency volume estimate agrees with the expected adult heart volume.

The presented method with spectrogram and inverse method show opportunities which are not present in other applications. The spectrogram is less sensitive to respiration than direct methods while the inverse methods incorporate additional signal information in the heart frequency and heart volume estimate. The inverse frequency method that combines the spectrogram with additional model information gives improved results.

When the at least one parameters have been optimized, heart model data can be generated based on the optimized values in combination with the used heart model. The heart model data can be represented to a user of the radar system, e.g. in a graphical way. The user of the system, e.g. a medical professional, is thereby provided with an image of the heart that is probed by the radar system. As an example, a beating heart image is shown following the performance of the human heart. Alternatively, the heart model data can be represented otherwise, e.g. using audible signals.

By generating heart model data using the optimized parameters, the heart performance can be monitored instantaneously in a contactless way, e.g. for monitoring patients having heart diseases, or patients suffering epileptic attacks. Specifically, the heart performance can be used as a measure for determining a chance of the occurrence of a future elliptic attack. Then, the quality of the heart performance can be determined. Also deviations in the heart performance can be detected.

According to an aspect of the invention, the processing unit is further arranged for filtering breathing effects from the received radar signal, thereby improving the parameter optimization process. Preferably, also background signals are removed. Advantageously, the heart performance can be monitored without requesting the person to temporally stop breathing. The process of filtering breathing effects can include determining breathing characteristics from the received radar data. As a side effect, both breathing and heart parameters can then be determined using the radar data.

A breathing frequency and a heart frequency can be determined using so-called direct methods wherein peaks in the spectral domain or in correlation functions are estimated, e.g. using a quadratically interpolated FFT (QIFFT) of Abe or with the centroid method of Massar.

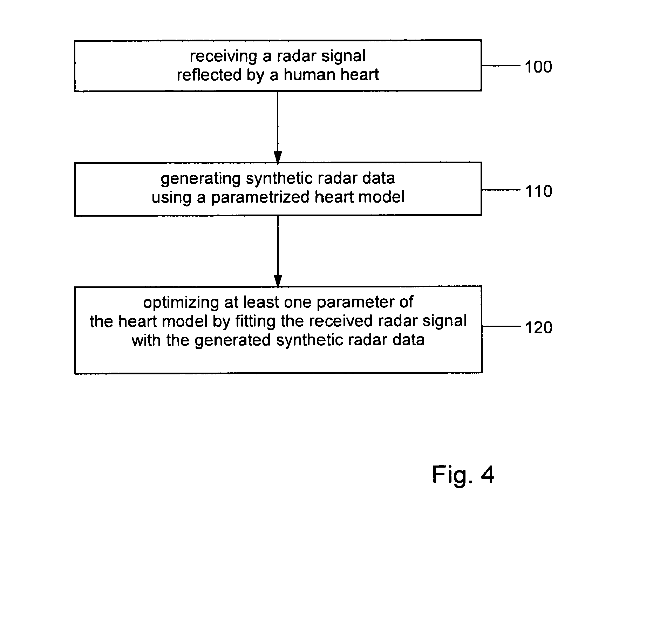

Figure 4 shows a flow chart of steps that are performed in a method according to the invention. The method is used for estimating parameters indicative of a heart performance. The method comprises a step of receiving (100) a radar signal reflected by a human heart, a step of generating (110) synthetic radar data using a parametrized heart model, and a step of optimizing (120) at least one parameter of the heart model by fitting the received radar signal with the generated synthetic radar data.

The method for estimating parameters indicative of a heart performance can be performed using dedicated hardware structures, such as FPGA and/or ASIC components. Otherwise, the method can also at least partially be performed using a computer program product comprising instructions for causing a processor to perform the above described steps of the method according to the invention. All steps can in principle be performed on a single processor. However it is noted that at least one step can be performed on a separate processor, e.g. the step of generating synthetic radar data using a parametrized heart model.

The invention is not restricted to the embodiments described herein. It will be understood that many variants are possible.

Instead of using three receivers also another number of receivers for receiving reflected radar signals can be used, e.g. more than three receivers, such as four or ten receivers, or less than three receivers, such as two receivers or a single receiver. Further, a multiple number of transmitters can be used to generate and transmit radar signals for reflection against the human heart. Apparently, a multiple number of receivers can be combined with a multiple number of transmitters to optimally obtain radar data of the heart.

Advantageously, a transmitter and a receiver can be implemented in a single radar station, thus optimally user radar devices. In addition, the transmitters and/or receivers can be located at different circumferential positions to gather information of the heart structure at different angles, thereby providing more accurate results and more information of heart parameters, such as local radial and tangential heart wall velocities.

The radar system according to the invention at least includes the processing unit 7 for performing the steps of generating synthetic radar data and optimizing at least one parameter of the heart model. The processing unit is thus arranged for processing radar data that is obtained via a radar equipment. During operation of the radar system, the radar equipment can be connected to the processing unit so that the processing steps can be performed in real time or in quasi real time. However, the radar data can also be provided at a later instant to the processing unit, e.g. using a data carrier, such as a DVD, carrying radar data that has been obtained by a remote radar equipment that is arranged for receiving a single or multiple radar signals reflected by a human heart, or using the Internet for transmitting radar data that has been measured remotely. Then, the radar system is not directly connected to a radar equipment.

It is noted that according to an aspect of the invention, the method can not be performed on radar data, but in principle also on other data that is obtained in a non-contact measurement, such as MRI data or acoustic data. The method includes then the steps of receiving a non-contact measurement signal that is physically associated to a human heart, generating synthetic non-contact measurement data using a parametrized heart model and optimizing at least one parameter of the heart model by fitting the received non-contact measurement signal with the generated synthetic non-contact data.

Other such variants will be apparent for the person skilled in the art and are considered to lie within the scope of the invention as defined in the following claims.

## Claims

1. A method for estimating parameters indicative of a heart performance, comprising the steps of:
- receiving a radar signal reflected by a human heart;
- generating synthetic radar data using a parametrized heart model; and
- optimizing at least one parameter of the heart model by fitting the received radar signal with the generated synthetic radar data.

2. A method according to claim 1, wherein the at least one optimized parameter includes a frequency, an amplitude and/or a phase of a heart beating signal.

3. A method according to claim 1 and/or 2, wherein the at least one optimized parameter includes geometrical dimensions of the heart.

4. A method according to any of the previous claims, wherein the parametrized heart model simulates a dynamic behaviour of the exterior heart surface.

5. A method according to any of the previous claims, wherein the radar signal is a frequency modulated continuous wave signal.

6. A method according to any of the previous claims, further including the step of generating heart model data based on the optimized at least one parameter.

7. A method according to any of the previous claims, wherein the fitting process is performed in the frequency or time domain.

8. A method according to any of the previous claims, further including filtering breathing effects from the received radar signal.

9. A radar system for estimating parameters indicative of a heart performance, comprising a processing unit that is arranged for performing the steps of:
- generating synthetic radar data using a parametrized heart model; and
- optimizing at least one parameter of the heart model by fitting a received radar signal, reflected by a human heart, with the generated synthetic radar data.

10. A system according to claim 9, further comprising a radar equipment for transmitting a radar signal towards a human heart and for receiving the radar signal reflected by the human heart.

11. A system according to claim 9 or 10, wherein the radar equipment includes a multiple number of transmitting units for transmitting radar signals from different directions towards the human heart.

12. A computer program product for estimating parameters indicative of a heart performance, which computer program product comprises instructions for causing a processor to perform the steps of:
- generating synthetic radar data using a parametrized heart model; and
- optimizing at least one parameter of the heart model by fitting a received radar signal, reflected by a human heart, with the generated synthetic radar data.
